# EUROPEAN PATENT APPLICATION

(11) **EP 4 338 663 A1**
(43) Date of publication of application: **20.03.2024**
(21) Application number: 22807365.6
(22) Date of filing: 27.04.2022
(51) Int. Cl.: A61B 5/02

(54) **PULSE WAVE SENSOR**

(30) Priority: 13.05.2021 JP 2021081688
(71) Applicant: Minebea Mitsumi Inc., Kitasaku-gun, Nagano 3890293 (JP)
(72) Inventor: EGUCHI, Kotaro, Kitasaku-gun, Nagano 389-0293 (JP); KASHINO, Takashi, Kitasaku-gun, Nagano 389-0293 (JP); ANJIKI, Syun, Kitasaku-gun, Nagano 389-0293 (JP); SHIMIZU, Ryo, Kitasaku-gun, Nagano 389-0293 (JP)
(74) Representative: Zabel, Julia Elisabeth
(86) International application number: PCT/JP2022/019201
(87) International publication number: WO 2022/239663

(57) **Abstract**

This pulse wave sensor has a strain body comprising a round opening part, and a strain gauge provided to the strain body that uses a Cr composite film as a resistor. If the diameter of the round opening part is d (mm), and the thickness of the strain body is t (mm): when the material of the strain body is SUS and d=32, 0.059≤t≤0.124; when the material is SUS and d=22, 0.046≤t≤0.099; when the material is SUS and d=13, 0.030≤t≤0.067; when the material is SUS and d=7, 0.026≤t≤0.034; when the material is copper and d=32, 0.084≤t≤0.166; when the material is copper and d=22, 0.066≤t≤0.132; when the material is copper and d=13, 0.044≤t≤0.088; when the material is copper and d=7, 0.032≤t≤0.050; when the material is aluminum and d=32, 0.097≤t≤0.212; when the material is aluminum and d=22, 0.079≤t≤0.168; when the material is aluminum and d=13, 0.050≤t≤0.107; and when the material is aluminum and d=7, 0.038≤t≤0.063. Pulse waves are detected on the basis of a change in a resistance value of the resistor accompanying deformation of the strain body.

## Description

### Technical Field

The present invention relates to a pulse wave sensor.

### Background Art

A pulse wave sensor for detecting arterial pulses generated when the heart pumps blood is known. An example of the pulse wave sensor is a pulse wave sensor provided with a pressure receiving plate as a strain generating body supported in a deflective manner by an action of an external force and a piezoelectric conversion element for converting the deflection of the pressure receiving plate into an electric signal. The pulse wave sensor has a dome-like shape in which a deflective region of the pressure receiving plate becomes a convex curved surface outward, and a pressure detecting element is provided on the inner surface of the top of the pressure receiving plate as the piezoelectric conversion element (for example, Patent Document 1).

### Citation List

### Patent document

[Patent Document 1] Japanese Unexamined Patent Application Publication No. 2002-78689

### Summary

### Problems to be solved by the Invention

Since a pulse wave sensor needs to detect a minute signal, a thin strain generating body is used to ensure the required sensitivity, but on the other hand, durability is also required. Therefore, the pulse wave sensor that is compatible with rigidity and durability is required. However, compatibility between sensitivity and rigidity has not been sufficiently investigated in conventional pulse wave sensors.

An object of the present invention is to provide a pulse wave sensor having both sensitivity and rigidity in view of the above.

### Means for Solving the Problem

A pulse wave sensor includes a strain generating body with a circular opening and a strain gauge provided on the strain generating body and including a Cr mixed phase film as a resistor, wherein a diameter of the circular opening is set to d [mm] and a thickness of the strain generating body is set to t [mm],wherein when a material of the strain generating body is SUS and the d is 32, a required range of t is 0.059≤t≤0.124; wherein when the material is SUS and the d is 22, a required range of t is 0.046≤t≤0.099; wherein when the material is SUS and the d is 13, a required range of t is 0.030≤t≤0.067; wherein when the material is SUS and the d is 7, a required range of t is 0.026≤t≤0.034; wherein when the material is copper and the d is 32, a required range of t is 0.084≤t≤0.166; wherein when the material is copper and the d is 22, a required range of t is 0.066≤t≤0.132; wherein when the material is copper and the d is 13, a required range of t is 0.044≤t≤0.088; wherein when the material is copper and the d is 7, a required range of t is 0.032≤t≤0.050; wherein when the material is aluminum and the d is 32, a required range of t is 0.097≤t≤0.212; wherein when the material is aluminum and the d is 22, a required range of t is 0.079≤t≤0.168; wherein when the material is aluminum and the d is 13, a required range of t is 0.050<t<0.107; wherein when the material is aluminum and the d is 7, a required range of t is 0.038<t<0.063; and wherein arterial pulses are detected based on a change in a resistance value of the resistor in response to a deformation of the strain generating body.

### Effect of Invention

According to the disclosed technique, the present invention can provide a pulse wave sensor having both sensitivity and rigidity.

### Brief Description of Drawings

FIG. 1 is a perspective view illustrating a pulse wave sensor according to a first embodiment;
FIG. 2 is a plan view illustrating the pulse wave sensor according to the first embodiment;
FIG. 3 is a cross-sectional view illustrating the pulse wave sensor of the first embodiment and illustrates a cross-section along the A-A line in FIG. 2;
FIG. 4 is a graph (No. 1) of an experimental result using SUS as a material for a strain generating body;
FIG. 5 is a graph (No. 2) of an experimental result using SUS as a material for a strain generating body;
FIG. 6 is a graph (No. 3) of an experimental result using SUS as a material for a strain generating body;
FIG. 7 is a graph (No. 4) of an experimental result using SUS as a material for a strain generating body;
FIG. 8 is a graph (No. 1) of an experimental result using copper as a material for a strain generating body;
FIG. 9 is a graph (No. 2) of an experimental result using copper as a material for a strain generating body;
FIG. 10 is a graph (No. 3) of an experimental result using copper as a material for a strain generating body;
FIG. 11 is a graph (No. 4) of an experimental result using copper as a material for a strain generating body;
FIG. 12 is a graph (No. 1) of an experimental result using aluminum as a material for a strain generating body;
FIG. 13 is a graph (No. 2) of an experimental result using aluminum as a material for a strain generating body;
FIG. 14 is a graph (No. 3) of an experimental result using aluminum as a material for a strain generating body;
FIG. 15 is a graph (No. 4) of an experimental result using aluminum as a material for a strain generating body;
FIG. 16 is a plan view illustrating a strain gauge according to the first embodiment; and
FIG. 17 is a cross-sectional view illustrating the strain gauge according to the first embodiment.

### Embodiment for carrying out the Invention

Hereinafter, the embodiments for carrying out the invention will be described with reference to the drawings. In each drawing, the same component parts are denoted by the same reference numerals, and duplicate descriptions may be omitted.

### <First Embodiment>

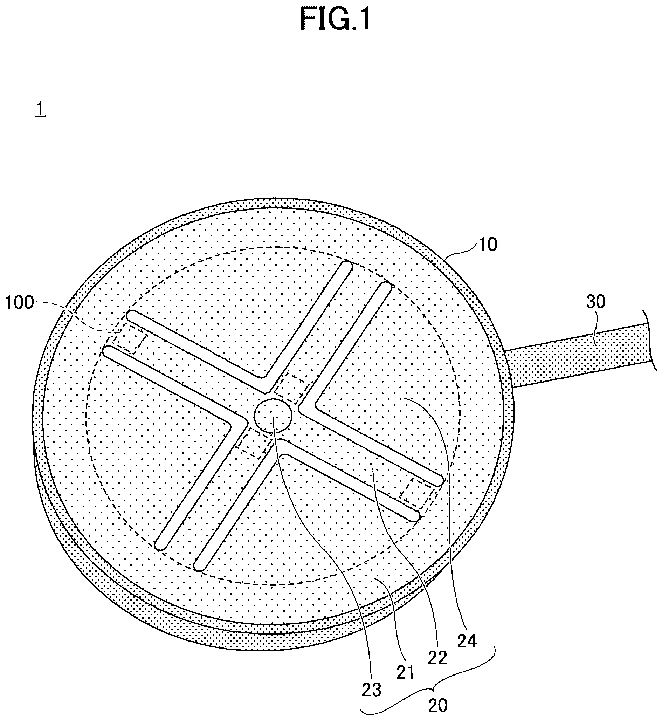

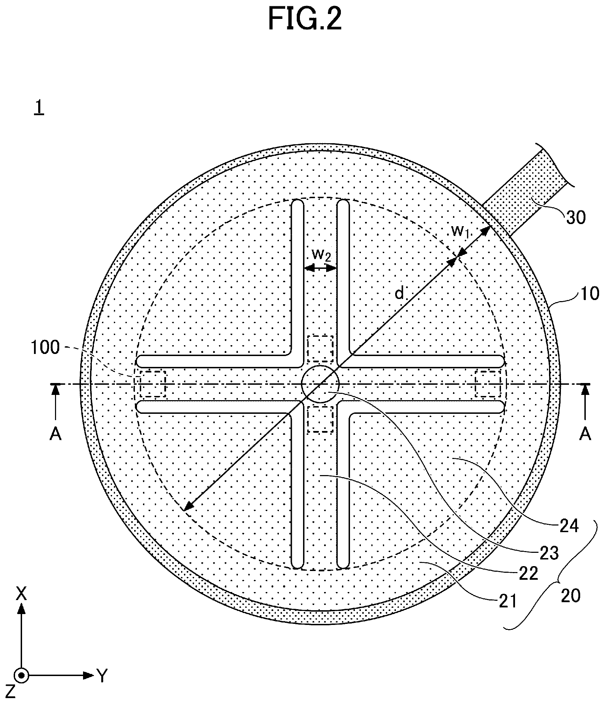

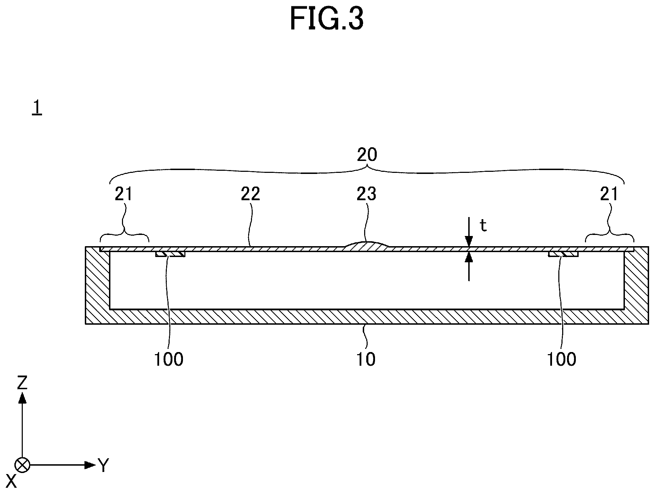

FIG. 1 is a perspective view illustrating a pulse wave sensor according to the first embodiment. FIG. 2 is a plan view illustrating the pulse wave sensor according to the first embodiment. FIG. 3 is a cross-sectional view illustrating the pulse wave sensor of the first embodiment and illustrates a cross-section along the A-A line in FIG. 2.

Referring to FIGS. 1 to 3, the pulse wave sensor 1 includes a housing 10, a strain generating body 20, a wire rod 30, and a strain gauge 100.

The strain generating body 20 includes a base portion 21, a beam portion 22, a load portion 23, and an extension portion 24. The strain generating body 20 has a flat plate shape, and each component is integrally formed by, for example, a pressing process and the like. The strain generating body 20 is, for example, four-fold symmetric in a plan view. The thickness t of the strain generating body 20 excluding the load portion 23 is constant. A suitable range of the thickness t will be described later.

In the present embodiment, for convenience, in the pulse wave sensor 1, the side where the load portion 23 of the strain generating body 20 is provided is the upper side or one side, and the side where the load portion 23 is not provided is the lower side or the other side. In addition, the surface where the load portion 23 of each part is provided is one surface or the upper surface, and the surface where the load portion 23 is not provided is the other surface or the lower surface. However, the pulse wave sensor 1 can be used in an inverted state or arranged at any angle. The plan view refers to the object viewed from the direction normal to the upper surface of the strain generating body 20, and the planar shape refers to the shape of the object viewed from the direction normal to the upper surface of the strain generating body 20.

In the pulse wave sensor 1, the housing 10 holds the strain generating body 20. The housing 10 has a hollow cylindrical shape, and the lower surface side is closed and the upper surface side is opened. The housing 10 can be formed of, for example, metal, resin, or the like. In order to close the opening on the upper surface side of the housing 10, a substantially disk-shaped strain generating body 20 is fixed by an adhesive or the like.

In the strain generating body 20, the base portion 21 is a circular frame-shaped (ring-shaped) region outside the circular dashed line illustrated in FIGS. 1 and 2. The region inside the circular dashed line may be called a circular opening. That is, the base portion 21 of the strain generating body 20 has the circular opening. The width w₁ of the base portion 21 is, for example, in a range from 1 mm to 5 mm. A suitable range of the inner diameter d (that is, the diameter of the circular opening) of the base portion 21 will be described later.

The beam portion 22 is provided so as to bridge the inside of the base portion 21. The beam portion 22 has, for example, two beams that cross each other in a cross shape in a plan view, and the region where the two beams cross includes a center of the circular opening. In the example of FIG. 2, one beam that forms a cross has its longitudinal direction in the X-direction, and the other beam that forms a cross has its longitudinal direction in the Y direction, and they are orthogonal to each other. Each of the two orthogonal beams is disposed inward relative to the inner diameter d (diameter of the circular opening) of the base portion 21 and is preferably as long as possible. That is, the length of each beam is preferably approximately equal to the diameter of the circular opening. In each beam constituting the beam portion 22, the width w₂ other than the region where the two beams cross is constant, for example, in a range from 1 mm to 5 mm. The width w₂ is not necessary constant. However, it is preferable in that the strain can be detected linearly by making the width w₂ constant.

The load portion 23 is provided on the beam portion 22. The load portion 23 is provided, for example, on a region where two beams constituting the beam portion 22 cross. The load portion 23 projects from the upper surface of the beam portion 22. The amount of projection of the load portion 23 relative to the upper surface of the beam portion 22 is, for example, about 0.1 mm. The beam portion 22 is flexible and elastically deformed when a load is applied to the load portion 23.

The four extension portions 24 are sectorial parts extending in the direction of the beam portion 22 from the inside of the base portion 21 in a plan view. A gap of about 1 mm is provided between each extension portion 24 and the beam portion 22. Since the extension portion 24 does not contribute to the sensing of the pulse wave sensor 1, the extension portion 24 may not be provided.

The wire rod 30 is a cable for inputting/outputting electric signals between the pulse wave sensor 1 and the outside. The wire rod 30 may be a shield cable, a flexible board, or the like.

The strain gauge 100 is provided on the strain generating body 20. The strain gauge 100 can be provided, for example, on the lower surface side of the beam portion 22. Since the beam portion 22 is a flat plate shape, the strain gauge can be easily attached to the beam portion 22. One or more strain gauges 100 may be provided, but in the present embodiment, four strain gauges 100 are provided. By providing four strain gauges 100, strain can be detected by a full bridge.

Two of the four strain gauges 100 are positioned on a portion of the beam close to the load portion 23 (center side of the circular opening) with an X-direction as a longitudinal direction, so that the two of the four strain gauges face each other across the load portion 23 in the plan view. The other two of the four strain gauges 100 are positioned on a portion of the beam close to the base portion 21 with a Y-direction as a longitudinal direction, so that the other two of the four strain gauges face each other across the load portion 23 in the plan view. With such an arrangement, compressive and tensile forces can be effectively detected and a large output can be obtained by the full bridge.

The pulse wave sensor 1 is fixed to an arm of a subject so that the load portion 23 is disposed above the radial artery of the subject. When a load is applied to the load portion 23 in response to the arterial pulses of the subject and the beam portion 22 is elastically deformed, the resistance value of the resistor of the strain gauge 100 changes. The pulse wave sensor 1 can detect the arterial pulses based on the change of the resistance value of the resistor of the strain gauge 100 caused by the deformation of the beam portion 22. The arterial pulses are, for example, output as periodic voltage changes from a measuring circuit connected with an electrode of the strain gauge 100.

In order for the pulse wave sensor 1 to detect the arterial pulses of the subject, the pulse wave sensor 1 is required to have both sensitivity and rigidity. In the pulse wave sensor 1, the required sensitivity is that the output of the strain gauge 100 is 0.1 mV/V or more when a load of 1 g is applied to the load portion 23. When the sensitivity is less than 0.1 mV/V, the arterial pulses cannot be clearly measured due to a decrease in the S/N ratio at the time of measurement. In addition, since a load of about 50 g is applied when the pulse wave sensor 1 is attached to the subject, the required rigidity is that the beam portion 22 does not cause plastic deformation when the load portion 23 is applied with the load of 50 g and a safety factor is set to 2. If this condition is not satisfied, it becomes difficult to use continuously as a pulse wave sensor.

The inventors found that the above sensitivity and rigidity can be compatible if the material of the strain generating body 20, the thickness t [mm], and the diameter d [mm] of the circular opening satisfy certain conditions. Specifically, the inventors experimentally determined the values of thickness t [mm] that can be compatible with the sensitivity and rigidity when the diameter d is 32 mm, 22 mm, 13 mm, and 7 mm using SUS (stainless steel), copper, and aluminum as the materials of the strain generating body 20.

The shape of the strain generating body 20 is as illustrated in FIGS. 2 and 3, and w₁ and w₂ are set to 3 mm. In addition, four strain gauges 100 using a Cr mixed phase film described later as a resistor are attached to the positions illustrated in FIGS. 2 and 3.

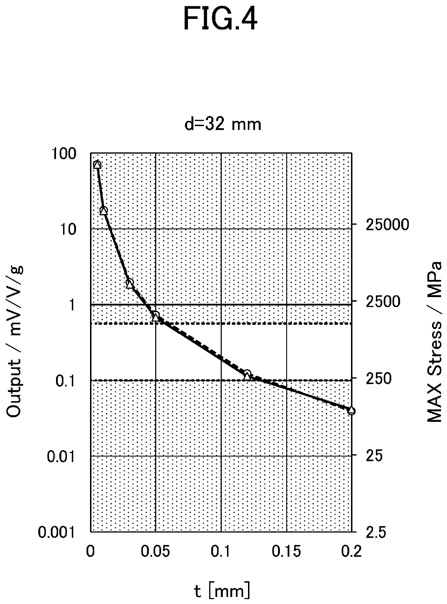

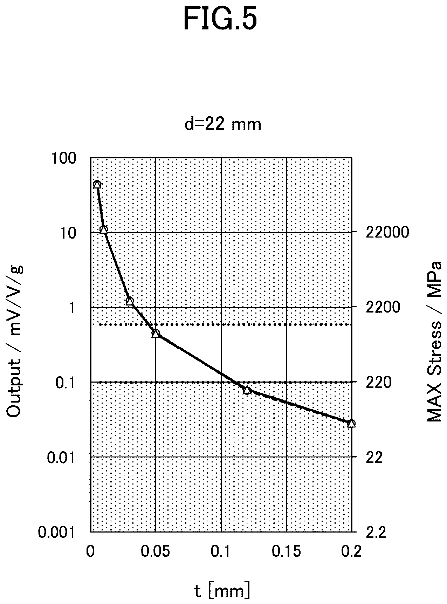

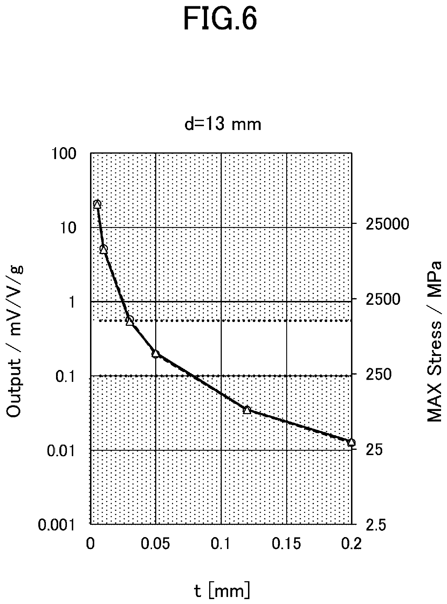

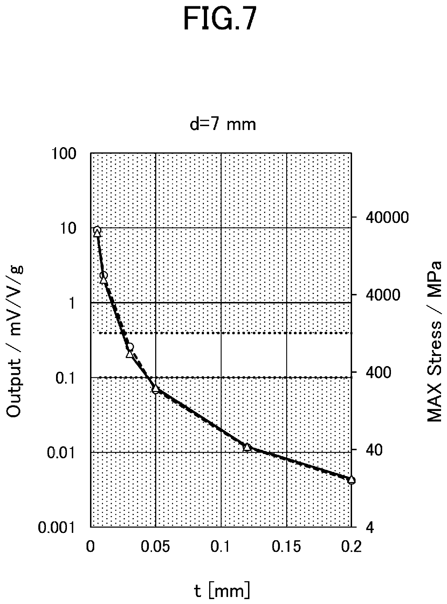

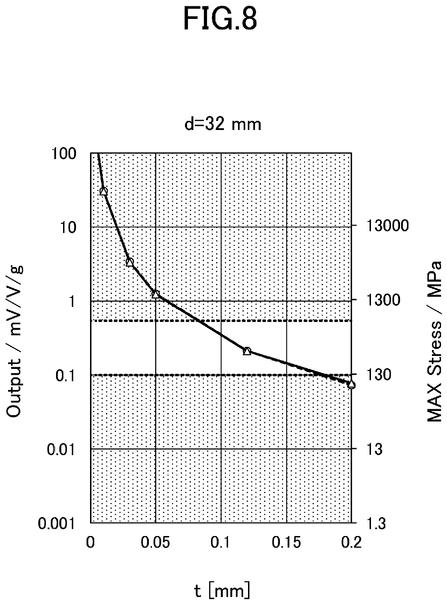

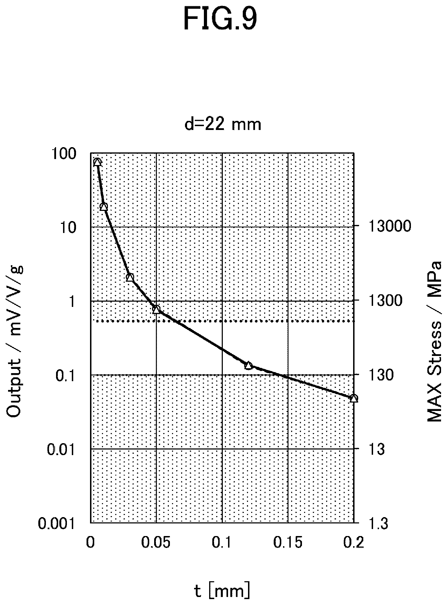

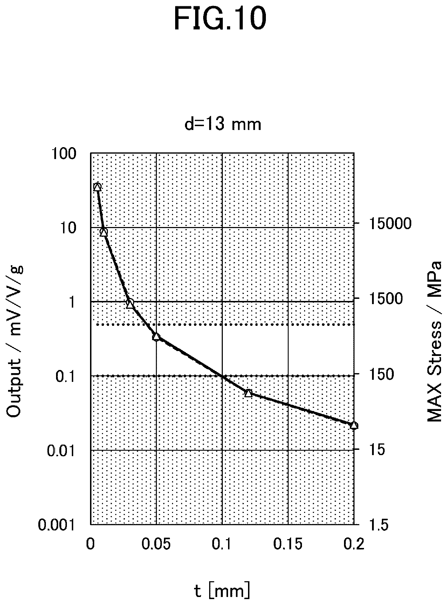

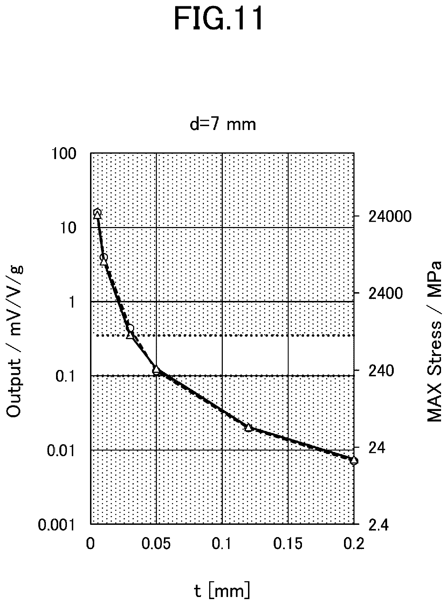

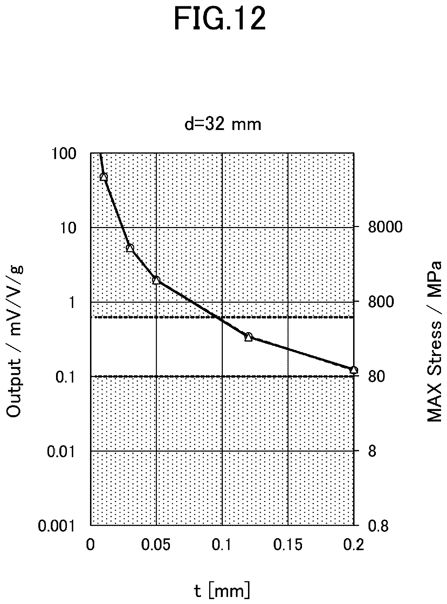

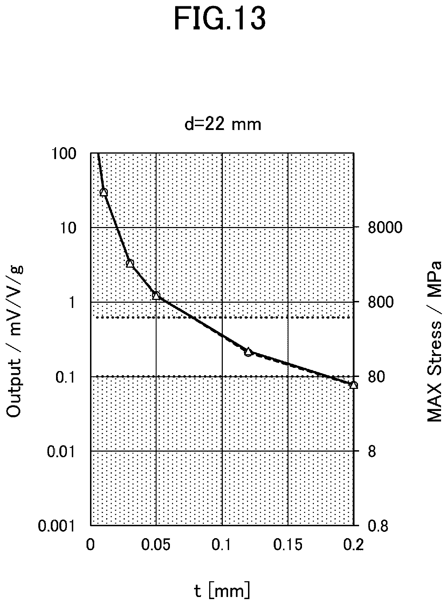

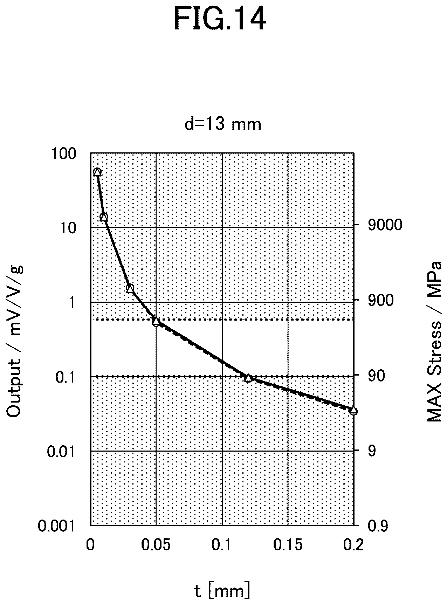

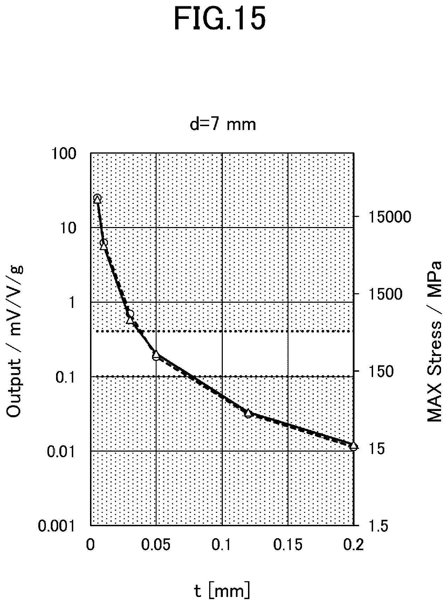

A graphical representation of the experimental results is illustrated in FIGS. 4 to 15. FIGS. 4 to 7 illustrate that SUS is used as the material of the strain generating body 20, FIGS. 8 to 11 illustrate that copper is used as the material of the strain generating body 20, and FIGS. 12 to 15 illustrate that aluminum is used as the material of the strain generating body 20. The left side of the vertical axis of each graph indicates the sensitivity, and the right side of the vertical axis indicates the maximum stress when a load of 50 g is applied to the load portion 23 and the safety factor is set to 2. The horizontal axis indicates the thickness t [mm] of the strain generating body 20.

In each graph, as the thickness t becomes thinner, the sensitivity becomes higher but the rigidity becomes lower, and the range of thickness t in which the sensitivity is 0.1 mV/V or more and the maximum stress is less than or equal to the yield strength (bearing capacity) is the range of thickness t in which the sensitivity and rigidity are compatible. In each graph, the range of thickness t in which the sensitivity and rigidity are compatible is the area indicated in white. The yield strength (bearing capacity) of SUS is 1275 MPa, that of copper is 675 MPa, and that of aluminum is 490 MPa.

**[Table 1]**

| Materials | d [mm] | tmin [mm] | Maximum Stress [Mpa] | tmax [mm] | Sensitivity [mV/V/g] |
|---|---|---|---|---|---|
| SUS | 32 | 0.059 | 1111 | 0.124 | 0.120 |
| | 22 | 0.046 | 1157 | 0.099 | 0.118 |
| | 13 | 0.030 | 1246 | 0.067 | 0.120 |
| | 7 | 0.026 | 909 | 0.034 | 0.148 |
| Copper | 32 | 0.084 | 553 | 0.166 | 0.116 |
| | 22 | 0.066 | 577 | 0.132 | 0.115 |
| | 13 | 0.044 | 662 | 0.088 | 0.121 |
| | 7 | 0.032 | 621 | 0.050 | 0.116 |
| Aluminum | 32 | 0.097 | 415 | 0.212 | 0.112 |
| | 22 | 0.079 | 405 | 0.168 | 0.112 |
| | 13 | 0.050 | 479 | 0.107 | 0.128 |
| | 7 | 0.038 | 449 | 0.063 | 0.116 |

Table 1 is a table that reads and summarizes the range of t that is compatible with sensitivity and rigidity. In Table 1, the diameter of the circular opening is d [mm] and the thickness of the strain generating body 20 is t [mm]. When the material of the strain generating body 20 is SUS and d is 32, the required range of t is 0.059≤t≤0.124 so as to have both sensitivity and rigidity. When the material of the strain generating body 20 is SUS and d is 22, the required range of t is 0.046≤t≤0.099. When the material of the strain generating body 20 is SUS and d is 13, the required range of t is 0.030≤t≤0.067. When the material of the strain generating body 20 is SUS and d is 7, the required range of t is 0.026≤t≤0.034.

When the material of the strain generating body 20 is copper and d is 32, the required range of t is 0.084≤t≤0.166. When the material of the strain generating body 20 is copper and d is 22, the required range of t is 0.066≤t≤0.132. When the material of the strain generating body 20 is copper and d is 13, the required range of t is 0.044≤t≤0.088. When the material of the strain generating body 20 is copper and d is 7, the required range of t is 0.032≤t≤0.050.

When the material of the strain generating body 20 is aluminum and d is 32, the required range of t is 0.097≤t≤0.212. When the material of the strain generating body 20 is aluminum and d is 22, the required range of t is 0.079≤t≤0.168. When the material of the strain generating body 20 is aluminum and d is 13, the required range of t is 0.050≤t≤0.107. When the material of the strain generating body 20 is aluminum and d is 7, the required range of t is 0.038≤t≤0.063.

Thus, if the material of the strain generating body 20, the thickness t [mm], and the diameter d [mm] of the circular opening satisfy certain conditions, the required sensitivity and rigidity can be compatible. In the case where the Cr mixed phase film is not used as the resistor of the strain gauge 100, the required sensitivity and rigidity cannot be compatible, or even if the sensitivity and rigidity are compatible, the allowable range of t becomes extremely narrow.

Hereinafter, the strain gauge 100 will be described.

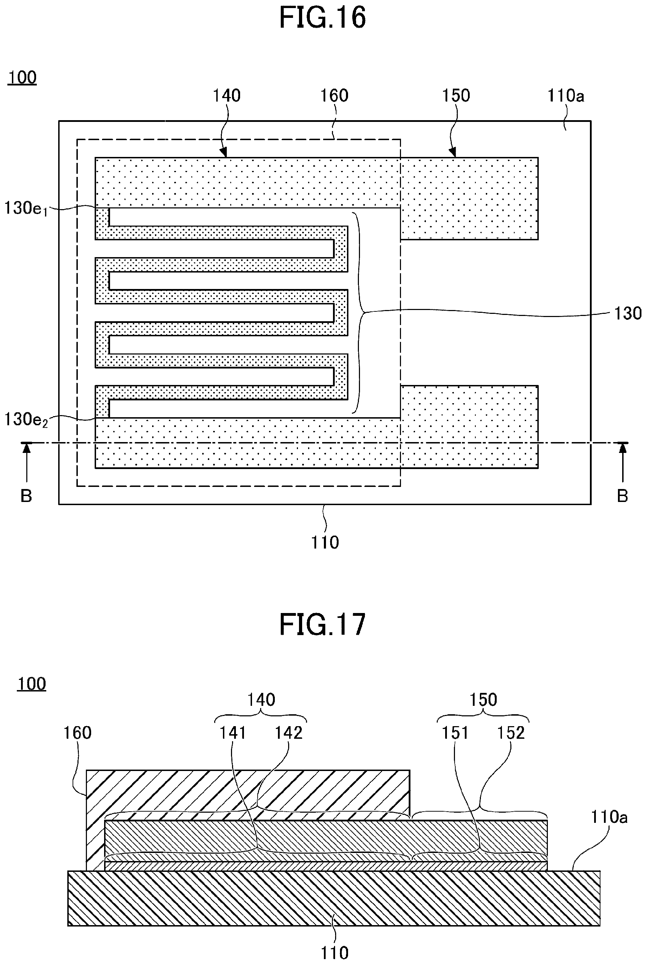

FIG. 16 is a plan view illustrating the strain gauge according to the first embodiment. FIG. 17 is a cross-sectional view illustrating a strain gauge according to the first embodiment, illustrating a cross-section along the line B-B of FIG. 16. Referring to FIGS. 16 and 17, the strain gauge 100 includes a substrate 110, a resistor 130, a wiring 140, an electrode 150, and a cover layer 160. In FIG. 16, only the outer edge of the cover layer 160 is illustrated as a dashed line for convenience. The cover layer 160 may be provided as necessary.

In FIGS. 16 and 17, for convenience, in the strain gauge 100, the side where the resistor 130 of the substrate 110 is provided is the upper side or one side, and the side where the resistor 130 is not provided is the lower side or the other side. In addition, the surface where the resistor 130 of each part is provided is one surface or the upper surface, and the surface where the resistor 130 is not provided is the other surface or the lower surface. However, the strain gauge 100 can be used in the reverse state or arranged at any angle. For example, in FIG. 2, the strain gauge 100 is attached to the beam portion 22 in the state of being upside down from FIG. 17. That is, the substrate 110 in FIG. 17 is attached to the lower surface of the beam portion 22 with an adhesive or the like. The plan view refers to an object viewed from the direction normal to the upper surface 110a of the substrate 110, and the planar shape refers to the object viewed from the direction normal to the upper surface 110a of the substrate 110.

The substrate 110 is a member that serves as a base layer for forming the resistor 130 and the like, and the substrate 110 has flexibility. The thickness of the substrate 110 is not particularly limited and can be appropriately selected according to the purpose, but can be, for example, in a range from 5 um to 500 um. In particular, when the thickness of the substrate 110 is in a range from 5 um to 200 um, it is preferable in terms of the transmissibility of strain from the surface of the strain generating body bonded to the lower surface of the substrate 110 via an adhesive layer or the like and also in terms of the dimensional stability with respect to the environment. When the thickness of the substrate 110 is 10 um or more, it is more preferable in terms of the insulating property.

The substrate 110 can be formed from, for example, an insulating resin film such as a polyimide (PI) resin, an epoxy resin, a polyetheretherketone (PEEK) resin, a polyethylene naphthalate (PEN) resin, a polyethylene terephthalate (PET) resin, a polyphenylene sulfide (PPS) resin, a liquid crystal polymer (LCP) resin, a polyolefin resin, and the like. The film refers to a member having a thickness of about 500 um or less and having flexibility.

The term "formed from an insulating resin film" here does not prevent the substrate 110 from containing fillers, impurities or the like in the insulating resin film. The substrate 110 may be formed from, for example, an insulating resin film containing a filler such as silica, alumina, or the like.

Materials other than the resin for the substrate 110 include, for example, crystalline materials such as SiO₂, ZrO₂ (including YSZ), Si, Si₂N₃, Al₂O₃ (including sapphire), ZnO, perovskite-based ceramics (CaTiOs, BaTiO₃), or the like, and further include amorphous glass or the like. Metals such as aluminum, aluminum alloy (duralumin), titanium, or the like may be used as materials for the substrate 110. In this case, for example, an insulating film is formed on the substrate 110 made of metal.

The resistor 130 is a thin film formed in a predetermined pattern on the substrate 110. The resistor 130 is a sensitive part that causes a resistance change when being strained. The resistor 130 may be formed directly on the upper surface 110a of the substrate 110, or through other layers on the upper surface 110a of the substrate 110. In FIG. 16, the resistor 130 is illustrated in a dark-colored pear-skin pattern for convenience.

The resistor 130 has a structure in which a plurality of thin and long portions is arranged at predetermined intervals in the longitudinal direction toward the same direction (the direction of the line B-B in FIG. 16), and the end portions of adjacent thin and long portions are mutually connected and folded back in a zigzag manner as a whole. The longitudinal direction of the plurality of thin and long portions is a grid direction, and the direction perpendicular to the grid direction is a grid width direction (the direction perpendicular to the line B-B in FIG. 16).

Two end portions of the thin and long portion in the longitudinal direction located on the outermost side of the grid width direction are bent in the grid width direction to form respective ends 130e₁ and 130e₂ in the grid width direction of the resistor 130. The respective ends 130e₁ and 130e₂ in the grid width direction of the resistor 130 are electrically connected to the electrodes 150 via a wiring 140. In other words, the wiring 140 electrically connects the respective ends 130e₁ and 130e₂ in the grid width direction of the resistor 130 with the respective electrodes 150.

The resistor 130 can be formed, for example, from a material containing chromium (Cr), a material containing nickel (Ni), or a material containing both Cr and Ni. That is, the resistor 130 can be formed from a material containing at least one of Cr and Ni. The material containing Cr includes, for example, a Cr mixed phase film. The material containing Ni includes, for example, copper nickel (Cu-Ni). The material containing both Cr and Ni includes, for example, nickel chromium (Ni-Cr).

Here, the Cr mixed phase film is a film in which Cr, CrN, Cr₂N, and the like are mixed. The Cr mixed phase film may contain unavoidable impurities such as chromium oxide and the like.

The thickness of the resistor 130 is not particularly limited and can be appropriately selected according to the purpose, but can be, for example, about in a range from 0.05 um to 2 um. In particular, when the thickness of the resistor 130 is 0.1 um or more, it is preferable in that the crystallinity (for example, the crystallinity of α-Cr) of the crystals constituting the resistor 130 is improved. When the thickness of the resistor 130 is 1 um or less, it is more preferable in that the film cracks caused by the internal stress of the film constituting the resistor 130 and warpage from the substrate 110 can be reduced. The width of the resistor 130 can be set to about in a range from 10 um to 100 um if the required specifications such as the resistance value and the lateral sensitivity are optimized and the wire breakage countermeasures are considered.

For example, when the resistor 130 is a Cr mixed phase film, the stability of the gauge characteristic can be improved by using alpha chromium (α-Cr), which is a stable crystalline phase, as the main component. Moreover, when the resistor 130 uses α-Cr as the main component, the gauge rate of the strain gauge 100 can be 10 or more, and the temperature coefficient of strain gauge rate TCS and the temperature coefficient of resistance TCR can be set within a range from - 1000 ppm/°C to +1000 ppm/°C. Here, the main component means that the target material occupies 50% by weight or more of the total material constituting the resistor, but from the viewpoint of improving the gauge characteristics, the resistor 130 preferably contains 80% by weight or more of α-Cr, and more preferably 90% by weight or more. α-Cr is Cr having a bcc structure (body-centered cubic lattice structure).

When the resistor 130 is a Cr mixed phase film, CrN and Cr₂N contained in the Cr mixed phase film are preferably 20% by weight or less. When CrN and Cr₂N contained in the Cr mixed phase film are 20% by weight or less, the lowering of the gauge ratio can be suppressed.

The proportion of Cr₂N in CrN and Cr₂N is preferably 80% by weight or more and less than 90% by weight, and more preferably 90% by weight or more and less than 95% by weight. When the proportion of Cr₂N in CrN and Cr₂N is 90% by weight or more and less than 95% by weight, the decrease in TCR (negative TCR) becomes more remarkable due to Cr₂N having semiconducting properties. In addition, by reducing ceramization, brittle fracture is reduced.

On the other hand, when a small amount of N₂ or atomic N is mixed or present in the film, N₂ or atomic N escapes from the film to the outside of the film due to an external environment (for example, a high-temperature environment), resulting in a change in film stress. By creating chemically stable CrN, a stable strain gauge without generating the unstable N can be obtained.

The wiring 140 is formed on the substrate 110 and is electrically connected to the resistor 130 and the electrodes 150. The wiring 140 has a first metal layer 141 and a second metal layer 142 laminated on the upper surface of the first metal layer 141. The wiring 140 is not limited to a straight line and can be formed in any pattern. The wiring 140 can be any width and any length. In FIG. 16, for convenience, the wiring 140 and the electrodes 150 are illustrated in a lighter-colored pear-skin pattern than the resistor 130.

The electrode 150 is formed on the substrate 110 and is electrically connected to the resistor 130 via the wiring 140, and is formed in a substantially rectangular shape, for example, wider than the wiring 140. The electrode 150 is a pair of electrodes for outputting a change in the resistance value of the resistor 130 caused by strain to the outside, and a lead wire or the like for external connection is joined, for example.

The electrode 150 has a pair of first metal layers 151 and a second metal layer 152 laminated on the upper surface of each first metal layer 151. The first metal layers 151 are electrically connected to the ends 130e₁ and 130e₂ of the resistor 130 through the first metal layers 141 of the wiring 140. The first metal layer 151 is formed in a substantially rectangular shape in a plan view. The first metal layer 151 may be formed in the same width as the wiring 140.

Although the resistor 130, the first metal layer 141, and the first metal layer 151 are designated separately for convenience, they can be integrally formed of the same material in the same process. Accordingly, the resistor 130, the first metal layer 141, and the first metal layer 151 have substantially the same thickness. In addition, although the second metal layer 142 and the second metal layer 152 are designated separately for convenience, they can be integrally formed of the same material in the same process. Therefore, the thickness of the second metal layer 142 and the second metal layer 152 is substantially the same.

The second metal layers 142 and 152 are formed from a material having a lower resistance than the resistor 130 (the first metal layers 141 and 151). The material of the second metal layers 142 and 152 is not particularly limited if the material has a lower resistance than the resistor 130, and can be appropriately selected according to the purpose. For example, if the resistor 130 is a Cr mixed phase film, the material of the second metal layers 142 and 152 may include Cu, Ni, Al, Ag, Au, Pt, or an alloy of any of these metals, a compound of any of these metals, or a laminated film suitably laminated with any of these metals, alloys, or compounds. The thickness of the second metal layers 142 and 152 is not particularly limited and may be suitably selected according to the purpose, but may be, for example, in a range from 3 um to 5 um.

The second metal layers 142 and 152 may be formed on a part of the upper surfaces of the first metal layers 141 and 151, or may be formed on the entire upper surfaces of the first metal layers 141 and 151. One or more other metal layers may be laminated on the upper surface of the second metal layer 152. For example, the second metal layer 152 may be a copper layer, and a gold layer may be laminated on the upper surface of the copper layer. Alternatively, the second metal layer 152 may be a copper layer, and a palladium layer and a gold layer may be successively laminated on the upper surface of the copper layer. By making the top layer of the electrode 150 a gold layer, the solder wettability of the electrode 150 can be improved.

As described above, the wiring 140 has a structure in which the second metal layer 142 is laminated on the first metal layer 141 made of the same material as the resistor 130. Therefore, since the wiring 140 has a lower resistance than the resistor 130, it is possible to suppress the wiring 140 from functioning as a resistor. As a result, the strain detection accuracy by the resistor 130 can be improved.

In other words, by providing the wiring 140 with a lower resistance than the resistor 130, the substantially sensitive portion of the strain gauge 100 can be limited to a local region where the resistor 130 is formed. Therefore, the strain detection accuracy by the resistor 130 can be improved.

In particular, in a highly sensitive strain gauge with a gauge ratio of 10 or more using a Cr mixed phase film as the resistor 130, limiting the substantially sensitive portion to the local region where the resistor 130 is formed by making the wiring 140 less resistant than the resistor 130 has a remarkable effect on improving the strain detection accuracy. In addition, making the wiring 140 less resistant than the resistor 130 also has an effect on reducing the lateral sensitivity.

The cover layer 160 is formed on the substrate 110, covers the resistor 130 and the wiring 140, and exposes the electrode 150. A portion of the wiring 140 may be exposed from the cover layer 160. By providing the cover layer 160 covering the resistor 130 and the wiring 140, mechanical damage or the like can be prevented from occurring to the resistor 130 and the wiring 140. Further, by providing the cover layer 160, the resistor 130 and the wiring 140 can be protected from moisture or the like. The cover layer 160 may be provided so as to cover the entire portion excluding the electrode 150.

The cover layer 160 may be formed of, for example, an insulating resin such as a PI resin, an epoxy resin, a PEEK resin, a PEN resin, a PET resin, a PPS resin, or a composite resin (for example, silicone resin, polyolefin resin). The cover layer 160 may contain fillers or pigments. The thickness of the cover layer 160 is not particularly limited and can be appropriately selected according to the purpose, but can be, for example, about in a range from 2 um to 30 um.

In order to manufacture the strain gauge 100, first, a substrate 110 is prepared and a metal layer (designated as a metal layer A for convenience) is formed on the upper surface 110a of the substrate 110. The metal layer A is a layer which is finally patterned into the resistor 130, the first metal layer 141, and the first metal layer 151. Accordingly, the material and thickness of the metal layer A are the same as those of the resistor 130, the first metal layer 141, and the first metal layer 151.

The metal layer A can be formed by, for example, a magnetron sputtering method by using a raw material capable of forming the metal layer A as a target. Instead of the magnetron sputtering method, the metal layer A may be formed by a reactive sputtering method, a vapor deposition method, an arc ion plating method, a pulsed laser deposition method, or the like.

From the viewpoint of stabilizing the gauge characteristics, a functional layer with a predetermined thickness may preferably be formed on the upper surface 110a of the substrate 110 by, for example, a conventional sputtering method before forming the metal layer A.

In the present invention, the functional layer refers to a layer having a function of promoting crystal growth of the metal layer A (resistor 130) which is at least an upper layer. In addition, the functional layer preferably has a function of preventing oxidation of the metal layer A by oxygen and moisture contained in the substrate 110 and a function of improving adhesion between the substrate 110 and the metal layer A. The functional layer may further have other functions.

Since the insulating resin film constituting the substrate 110 contains oxygen and moisture, especially when the metal layer A contains Cr, Cr forms an auto-oxide film. Therefore, providing the functional layer to prevent oxidation of the metal layer A is effective.

The material of the functional layer is not particularly limited and can be suitably selected according to the purpose as long as the material has a function of promoting crystal growth of at least the metal layer A (resistor 130) which is at least an upper layer. Examples of the material of the functional layer include metals selected from the group consisting of chromium (Cr), titanium (Ti), vanadium (V), niobium (Nb), tantalum (Ta), nickel (Ni), yttrium (Y), zirconium (Zr), hafnium (Hf), silicon (Si), carbon (C), zinc (Zn), copper (Cu), bismuth (Bi), iron (Fe), molybdenum (Mo), tungsten (W), ruthenium (Ru), rhodium (Rh), rhenium (Re), osmium (Os), iridium (Ir), platinum (Pt), palladium (Pd), silver (Ag), gold (Au), cobalt (Co), manganese (Mn), and aluminum (Al). One type of the metal selected from the group or two or more types of the metal selected from the group can be used. An alloy of any metals selected from the group or a compound of any metals selected from the group may also be used.

Examples of the alloys include FeCr, TiAl, FeNi, NiCr, CrCu, and the like. Examples of the compounds include TiN, TaN, Si₃N₄, TiO₂, Ta₂O₅, SiO₂, and the like.

When the functional layer is formed from a conductive material such as a metal or alloy, the thickness of the functional layer is preferably 1/20 or less of the thickness of the resistor. In such a range, crystal growth of α-Cr can be promoted, and a part of the current flowing through the resistor flows to the functional layer, thereby preventing the strain detection sensitivity from being reduced.

When the functional layer is formed from a conductive material such as a metal or alloy, the thickness of the functional layer is preferably 1/50 or less of the thickness of the resistor. In such a range, the crystal growth of α-Cr can be promoted, and a part of the current flowing in the resistor flows in the functional layer, thereby further preventing the detection sensitivity of the strain from being lowered.

When the functional layer is formed from a conductive material such as a metal or alloy, the thickness of the functional layer is more preferably 1/100 or less of the thickness of the resistor. In such a range, a part of the current flowing in the resistor flows in the functional layer, thereby further preventing the detection sensitivity of the strain from being lowered.

When the functional layer is formed from an insulating material such as an oxide or nitride, the thickness of the functional layer is preferably in a range from 1 nm to 1 um. In this range, crystal growth of α-Cr can be promoted, and the film can be easily formed without cracks in the functional layer.

When the functional layer is formed from an insulating material such as an oxide or nitride, the thickness of the functional layer is more preferably in a range from 1 nm to 0.8 um. In this range, crystal growth of α-Cr can be promoted and the film can be formed more easily without cracks in the functional layer.

If the functional layer is formed from an insulating material such as an oxide or nitride, the thickness of the functional layer is more preferably in a range from 1 nm to 0.5 um. In this range, crystal growth of α-Cr can be promoted and the film can be formed more easily without cracks in the functional layer.

The planar shape of the functional layer is patterned almost identically to the planar shape of the resistor illustrated in FIG. 16, for example. However, the planar shape of the functional layer is not limited to the case where it is almost identical to the planar shape of the resistor. In the case where the functional layer is formed from an insulating material, it is not necessary to pattern it in the same shape as the planar shape of the resistor. In this case, the functional layer may be formed in a solid shape at least in the region where the resistor is formed. Alternatively, the functional layer may be formed in a solid pattern over the entire upper surface of the substrate 110.

Further, when the functional layer is formed from an insulating material, the thickness of the functional layer is formed relatively thick, in a range from 50 nm to 1 um, and in a solid pattern, which increases the thickness and surface area of the functional layer, allowing the heat generated by the resistor to dissipate to the substrate 110. As a result, in the strain gauge 100, a decrease in measurement accuracy due to self-heating of the resistor can be suppressed.

For example, the functional layer can be formed in vacuum by a conventional sputtering method in which argon (Ar) gas is introduced into a chamber by using a raw material capable of forming the functional layer as a target. By using the conventional sputtering method, the functional layer is formed while etching the upper surface 110a of the substrate 110 with Ar, so that the adhesion improvement effect can be obtained by minimizing the film formation amount of the functional layer.

However, this is an example of the film formation method of the functional layer, and the functional layer may be formed by other methods. For example, the adhesion improvement effect may be obtained by activating the upper surface 110a of the substrate 110 by plasma treatment using Ar or the like before the film formation of the functional layer, and then the functional layer may be formed in vacuum by a magnetron sputtering method.

The combination of the material of the functional layer and the material of the metal layer A is not particularly limited and can be appropriately selected according to the purpose, but, for example, a Cr mixed phase film using Ti as the functional layer and α-chromium (α-Cr) as the main component of the metal layer A can be formed.

In this case, for example, the metal layer A can be formed by a magnetron sputtering method in which Ar gas is introduced into the chamber by using a raw material capable of forming a Cr mixed phase film as a target. Alternatively, the metal layer A can be formed by a reactive sputtering method in which an appropriate amount of nitrogen gas is introduced into the chamber together with Ar gas by using pure Cr as a taeget. At this time, the proportion of CrN and Cr₂N contained in the Cr mixed phase film and the proportion of Cr₂N in CrN and Cr₂N can be adjusted by changing the amount of nitrogen gas introduced and the pressure (nitrogen partial pressure) or by adjusting the heating temperature by providing a heating step.

In these methods, the growth surface of the Cr mixed phase film is determined by the functional layer formed from Ti, and the Cr mixed phase film mainly composed of α-Cr, which is a stable crystal structure, can be formed. In addition, the Ti constituting the functional layer diffuses into the Cr mixed phase film, thereby improving the gauge characteristics. For example, the gauge rate of the strain gauge 100 may be 10 or more, and the temperature coefficient of strain gauge TCS and the temperature coefficient of resistance TCR may be within a range from -1000 ppm/°C to +1000 ppm/°C. When the functional layer is formed from Ti, Ti or titanium nitride (TiN) may be included in the Cr mixed phase film.

In the case where the metal layer A is the Cr mixed phase film, the functional layer formed from Ti has a function of promoting crystal growth of the metal layer A, a function of preventing oxidation of the metal layer A by oxygen and moisture contained in the substrate 110, and a function of improving adhesion between the substrate 110 and the metal layer A. The same applies when Ta, Si, Al, and Fe are used instead of Ti as the functional layer.

Thus, by providing the functional layer in the lower layer of the metal layer A, the crystal growth of the metal layer A can be promoted, and the metal layer A composed of the stable crystal phase can be produced. As a result, the stability of the gauge characteristics can be improved in the strain gauge 100. Further, by diffusing the material constituting the functional layer into the metal layer A, the gauge characteristics can be improved in the strain gauge 100.

Next, the second metal layer 142 and the second metal layer 152 are formed on the upper surface of the metal layer A. The second metal layer 142 and the second metal layer 152 can be formed by, for example, a photolithography method.

Specifically, a seed layer is first formed to cover the upper surface of the metal layer A by, for example, a sputtering method or an electroless plating method. Next, a photosensitive resist is formed on the entire surface of the upper surface of the seed layer, and an opening is formed to expose an area for forming the second metal layer 142 and the second metal layer 152 by exposure and development. At this time, the pattern of the second metal layer 142 can be formed so as to be an arbitrary shape by adjusting the shape of the opening of the resist. For example, a dry film resist or the like can be used as the resist.

Next, for example, a second metal layer 142 and a second metal layer 152 are formed on the seed layer exposed in the opening by an electrolytic plating method using the seed layer as a power supply path. The electrolytic plating method is suitable in that the tact is high and a low-stress electrolytic plating layer can be formed as the second metal layer 142 and the second metal layer 152. By making the thick electrolytic plating layer low-stress, warping of the strain gauge 100 can be prevented. The second metal layer 142 and the second metal layer 152 may be formed by a non-electrolytic plating method.

Next, the resist is removed. The resist can be removed, for example, by dipping the resist material into a dissolvable solution.

A photosensitive resist is then formed on the entire surface of the upper surface of the seed layer, exposed and developed, and patterned into a planar shape similar to that of the resistor 130, wiring 140, and electrode 150 of FIG. 16. For example, a dry film resist and the like can be used as the resist. Then, using the resist as an etching mask, the metal layer A and the seed layer exposed from the resist are removed to form the planar resistor 130, the wiring 140, and the electrode 150 illustrated in FIG. 16.

For example, unnecessary portions of the metal layer A and the seed layer can be removed by wet etching. If a functional layer is formed under the metal layer A, the functional layer is patterned by etching into the planar shape illustrated in FIG. 16 in the same manner as the resistor 130, the wiring 140, and the electrode 150. At this point, a seed layer is formed on the resistor 130, the first metal layer 141, and the first metal layer 151.

Next, the second metal layer 142 and the second metal layer 152 are formed by using the second metal layer 142 and the second metal layer 152 as etching masks and removing unnecessary seed layers exposed from the second metal layer 142 and the second metal layer 152. The seed layers immediately below the second metal layer 142 and the second metal layer 152 remain. For example, the unnecessary seed layer can be removed by wet etching using an etchant in which the seed layer is etched and the functional layer, the resistor 130, the wiring 140, and the electrode 150 are not etched.

Then, if necessary, a cover layer 160 covering the resistor 130 and the wiring 140 and exposing the electrode 150 is provided on the upper surface 110a of the substrate 110 to form the strain gauge 100. For example, the cover layer 160 can be formed by laminating a semi-cured thermosetting insulating resin film on the upper surface 110a of the substrate 110 so as to cover the resistor 130 and the wiring 140 and expose the electrode 150, and curing the film by heating. The cover layer 160 can be prepared by coating the upper surface 110a of the substrate 110 with a liquid or paste thermosetting insulating resin covering the resistor 130 and the wiring 140 and exposing the electrode 150, and heating to cure the film.

Although the preferred embodiments have been described in detail above, they are not limited to the embodiments described above, and various modifications and substitutions can be made to the embodiments described above without departing from the scope described in the claims.

The present application is based on and claims priority of Patent Application No. 2021-081688, filed May 13, 2021 with the Japan Patent Office, the entire contents of Japanese Patent Application No. 2021-081688 are hereby incorporated by reference.

### Description of Sign

- 1: Pulse wave sensor
- 10: Housing
- 20: Strain generating body
- 21: Base portion
- 22: Beam portion
- 23: Load portion
- 24: Extension portion
- 30: Wire rod
- 100: Strain gauge
- 110: Substrate
- 110a: Upper surface
- 130: Resistor
- 140: Wiring
- 150: Electrode
- 160: Cover layer
- 130e₁, 130e₂: Ends

## Claims

1. A pulse wave sensor comprising:
a strain generating body with a circular opening; and
a strain gauge provided on the strain generating body and including a Cr mixed phase film as a resistor,
wherein a diameter of the circular opening is set to d [mm] and a thickness of the strain generating body is set to t [mm],
wherein when a material of the strain generating body is SUS and the d is 32, a required range of t is 0.059≤t≤0.124;
wherein when the material is SUS and the d is 22, a required range of t is 0.046≤t≤0.099;
wherein when the material is SUS and the d is 13, a required range of t is 0.030≤t≤0.067;
wherein when the material is SUS and the d is 7, a required range of t is 0.026≤t≤0.034;
wherein when the material is copper and the d is 32, a required range of t is 0.084≤t≤0.166;
wherein when the material is copper and the d is 22, a required range of t is 0.066≤t≤0.132;
wherein when the material is copper and the d is 13, a required range of t is 0.044≤t≤0.088;
wherein when the material is copper and the d is 7, a required range of t is 0.032≤t≤0.050;
wherein when the material is aluminum and the d is 32, a required range of t is 0.097≤t≤0.212;
wherein when the material is aluminum and the d is 22, a required range of t is 0.079≤t≤0.168;
wherein when the material is aluminum and the d is 13, a required range of t is 0.050<t<0.107;
wherein when the material is aluminum and the d is 7, a required range of t is 0.038<t<0.063; and
wherein arterial pulses are detected based on a change in a resistance value of the resistor in response to a deformation of the strain generating body.

2. A pulse wave sensor comprising:
a strain generating body; and
a strain gauge provided on the strain generating body and including a Cr mixed phase film as a resistor,
wherein the strain generating body includes:
a base portion with a circular opening;
a beam portion bridging an inside of the base portion; and
a load portion provided on the beam portion,
wherein the strain generating body is in a form of a flat plate, and
wherein arterial pulses are detected based on a change in a resistance value of the resistor in response to a deformation of the strain generating body.

3. The pulse wave sensor according to claim 1, wherein the strain generating body includes:
a base portion with the circular opening;
a beam portion bridging an inside of the base portion; and
a load portion provided on the beam portion.

4. The pulse wave sensor according to claim 3, wherein the strain generating body is in a form of a flat plate.

5. The pulse wave sensor according to any one of claims 2 to 4,
wherein the beam portion has two beams that cross each other in a cross shape in a plan view,
wherein a region where the two beams cross includes a center of the circular opening, and
wherein the load portion is provided on the region where the two beams cross.

6. The pulse wave sensor according to claim 5,
wherein the pulse wave sensor includes four strain gauges,
wherein two of the four strain gauges are positioned on a portion of the beam close to the load portion with a first direction as a longitudinal direction, so that the two of the four strain gauges face each other across the load portion in the plan view, and
wherein the other two of the four strain gauges are positioned on a portion of the beam close to the base portion with a second direction perpendicular to the first direction as a longitudinal direction, so that the other two of the four strain gauges face each other across the load portion in the plan view.

7. The pulse wave sensor according to claim 5 or 6, wherein a length of each of the beams is approximately equal to the diameter of the circular opening.

8. The pulse wave sensor according to any one of claims 5 to 7, wherein a width other than the region where the two beams cross is constant in each of the beams.
